(19) **Europäisches Patentamt / European Patent Office / Office européen des brevets**

(11) **EP 1 688 742 B1**

(12) **EUROPEAN PATENT SPECIFICATION**

(45) Date of publication and mention of the grant of the patent:
**15.04.2009 Bulletin 2009/16**

(51) Int Cl.:
*G01N 33/50* (2006.01)    *C12Q 1/32* (2006.01)
*G01N 33/66* (2006.01)

(21) Application number: **06002187.0**

(22) Date of filing: **02.02.2006**

(54) **Electrochemical biosensor**

Elektrochemischer Biosensor

Biocapteur électrochimique

(84) Designated Contracting States:
**AT BE BG CH CY CZ DE DK EE ES FI FR GB GR HU IE IS IT LI LT LU LV MC NL PL PT RO SE SI SK TR**

(30) Priority: **04.02.2005 KR 2005010720**

(43) Date of publication of application:
**09.08.2006 Bulletin 2006/32**

(73) Proprietor: **i-SENS, INC.**
**Seoul 139-845 (KR)**

(72) Inventors:
• **Cui, Gang**
  **Seoul 139-845 (KR)**
• **Yoo, Jae Hyun**
  **Seoul 133-804 (KR)**
• **Kim, Moon Hwan**
  **Youngdeungpo-ku**
  **Seoul 150-867 (KR)**
• **Kim, Ju Yong**
  **Suwon-si**
  **Gyeonggi-do 440-300 (KR)**
• **Lee, Joung Su**
  **Seongbuk-gu**
  **Seoul 136-784 (KR)**
• **Kim, Keun Ki**
  **Seoul 132-042 (KR)**
• **Nam, Hakhyun**
  **Kangbuk-gu**
  **Seoul 142-063 (KR)**
• **Cha, Geun Sig**
  **Seoul 120-841 (KR)**

(74) Representative: **Koepe, Gerd L.**
**Koepe & Partner**
**Robert-Koch-Strasse 1**
**80538 München (DE)**

(56) References cited:
**EP-A- 1 486 778          US-A1- 2004 045 821**

• **CUI GANG ET AL: "Differential thick-film amperometric glucose sensor with an enzyme-immobilized nitrocellulose membrane" ELECTROANALYSIS, vol. 13, no. 3, March 2001 (2001-03), pages 224-228, XP002382013 ISSN: 1040-0397**
• **GANG CUI ET AL: "Disposable amperometric glucose sensor electrode with enzyme-immobilized nitrocellulose strip" TALANTA, ELMSFORD,NY, US, vol. 54, 2001, pages 1105-1111, XP002972399**

## Description

FIELD OF THE INVENTION

The present invention relates to a method for measuring blood glucose levels using an electrochemical biosensor.

BACKGROUND OF THE INVENTION

For the diagnosis and prophylaxis of diabetes mellitus, the importance of periodic monitoring of blood glucose levels has been increasingly emphasized. Nowadays, strip-type biosensors designed for hand-held reading devices, which are usually based on colorimetry or electrochemistry, allow individuals to readily monitor glucose levels in blood.

The electrochemistry applied to biosensors is explained by the following Reaction Formula I, featuring the use of an electron transfer mediator. Examples of the electron transfer mediator include: ferrocene and derivatives thereof; quinines and derivatives thereof; transition metal containing organic or inorganic compounds, such as hexamine ruthenium, osmium-containing polymers, potassium ferricyanide, etc.; organic conducting salts; and viologen.

$$\text{Reaction Formula I} \qquad \text{Glucose} + \text{GOx-FAD} \rightarrow \text{gluconic acid} + \text{GOx-FADH}_2 \quad \text{GOx-FADH}_2 + M_{ox} \rightarrow \text{GOx-FAD} + M_{red}$$

wherein, GOx represents glucose oxidase; GOx-FAD and GOx-FADH$_2$ respectively represent an oxidized and a reduced state of glucose-associated FAD (flavin adenine dinucleotide), a cofactor required for the catalysis of glucose oxidase; and $M_{ox}$ and $M_{red}$ denote an oxidized and a reduced state of an electron transfer mediator, respectively.

As shown in Reaction Formula I, (1) glucose in blood is oxidized to gluconic acid by the catalysis of the glucose oxidase, with the cofactor FAD reduced to FADH$_2$. (2)Then, the reduced cofactor FADH$_2$ transfers electrons to the mediator, so that FADH$_2$ returns to its oxidized state, that is FAD; and the mediator is reduced. The reduced mediator is diffused to the surface of the electrodes. The series of reaction cycles is driven by the anodic potential applied at the working electrode, and the redox current proportional to the level of glucose is measured.

Compared to biosensors based on colorimetry, the electrochemical biosensors (e.g., based on electrochemistry) has the advantages of being not influenced by oxygen and allowing the use of samples, even if cloudy, without pretreatment thereof.

The document G. Cui et al., Electroanalysis 2001 (13), 224 to 228, discloses a differential thick-film amperometric Glucose sensor with an enzyme-immobilized nitrocellulose membrane, wherein hexacyanoferrate (III) is used as the mediator.

The document G. Cui et al., Talanta 54 : 1105 to 1111 (2001), discloses a disposable amperometric glucose sensor electrode with an enzyme-immobilized nitrocellulose strip, wherein hexamineruthenium (III) chloride is used as the mediator.

Electrochemical biosensors, although convenient for monitoring and controlling blood glucose levels, have accuracies depending greatly on the presence of various readily oxidizable species, such as ascorbic acid, acetaminophen, uric acid, etc., in blood samples.

Another serious measurement bias results from blood hematocrits (measure of the volume of red blood cells as a percentage of the total blood volume). A large hematocrit level-dependent bias may lead to an erroneous judgment for those who must regularly monitor their blood glucose levels with disposable biosensor strips, possibly causing even loss of life.

Methods have been suggested for reducing the measurement bias from blood hematocrits, including the use of additional hematocrit separation; or an erythrocyte exclusion layer dispensed on the reagent layer (JP 1134461, JP 2000338076, and US 5658444); screen-printable reagent/blood separation paste formulated with a silica filler (US 6241862 B1), and a chemometric correction method combined with double excitation potentials (WO 01/57510 A2).

These disclosed methods, however, have difficulty in dispensing reagent cocktails on a working electrode, requiring either extra steps for the manufacturing process or a large loss of reagents in printing a reagent layer.

It is very important to accurately measure glucose levels in a small blood sample within a short time using a biosensor in terms of the convenience of the users. An accurate measure from a small volume of a sample, less than 1 $\mu$l, preferably 0.5$\mu$ l, or more preferably 0.3 $\mu$l, makes it possible to use any body region, for example, the forearm, as a blood sampling site, thereby greatly reducing the patient's pain upon blood sampling.

The response time period for measurement is preferably within 10 sec, more preferably within 5 sec, and most preferably within 3 sec. However, it is almost impossible to achieve this desirable goal using the techniques known thus far.

An electrochemical biosensor suitable for such measurements was proposed in the document EP-A 1 186 778 which comprises a reagent layer composition that can substantially reduce the measurement bias arising from hematocrits, and another one in the document US-A 2004/004,821, as comprising a sample introducing part including a sample introducing passage, an air discharge passage and a void.

SUMMARY OF THE INVENTION

It is therefore an object of present invention to provide a method for measuring blood glucose levels using a biosensor, which can remarkably reduce the measurement bias arising from hematocrits on the basis of the information about sample fluidity.

It is another object of the present invention to provide a method for measuring blood glucose levels using a biosensor, which decrease the possibility of obtaining false information by excluding the blood sample of abnormally high or low fluidity.

It is a further object of the present invention to provide a method for measuring blood glucose levels using a biosensor, which can detect a change in blood sampling speed caused by the aging of the biosensor, thereby providing the information about quality control upon manufacture.

It is still a further object of the present invention to provide a method for measuring blood glucose levels using a biosensor, which allows a blood sample to be introduced fast and constantly, without pretreatment and to be analyzed for blood glucose levels accurately and fast.

The above objects could be accomplished by the provision of a method for measuring blood glucose levels, using an electrochemical biosensor provided with a converse-type thin layer electrochemical cell, said converse-type thin layer electrochemical cell comprising: a working electrode formed on a flat insulating substrate; an auxiliary electrode formed on a separate flat insulating substrate so as to face the working electrode; a fluidity-determining electrode, formed at a predetermined distance from the working electrode on the flat insulating substrate used for the working electrode or the auxiliary electrode; an adhesive spacer, provided with a sample-introducing part having a micro-passage, for spatially separating the working electrode and the auxiliary electrode by being interposed therebetween; an electrode connector, printed with a thick conductive material on a portion of the auxiliary electrode, for three-dimensionally connecting the working electrode to the auxiliary electrode; and a reagent layer containing an electron transfer mediator and an oxidation enzyme, said method comprising the steps of: (1) introducing a blood sample into a sensor strip-inserted reading device; (2) applying predetermined respective potential differences between the working electrode and the auxiliary electrode and between the fluidity-determining electrode and the auxiliary electrode; (3) causing a first change in current between the working electrode and the auxiliary electrode so as to allow these electrodes to have the same voltage, as the blood sample is introduced; (4) detecting the flow of the blood sample with the fluidity-determining electrode to cause a second change in current between the auxiliary electrode and the fluidity determining electrode to adjust voltages between the auxiliary electrode and the fluidity-determining electrode into the same value, thereby providing information about the time difference from the change detected by the working electrode; (5) sufficiently mixing the reagent layer with the blood sample to apply a predetermined voltage between the working electrode and the auxiliary electrode to cause cycling reactions within the converse-type thin layer electrochemical cell; and (6) determining the level of glucose in the blood sample on the basis of the time information obtained in the step (4) and the steady-state current obtained in the step (5).

In the method, the blood sample of the step (1) ranges in volume from 0.1 to 0.7 $\mu$l and introduced into the sensor strip without being pretreated.

In the method, the potential differences of the step (2) are caused by an electrical change between the working electrode and the auxiliary electrode and between the fluidity-determining electrode and the auxiliary electrode upon applying a direct current, a low- or high-frequency alternating current, a high impedance, or a pulse selected from among square waves, pyramidal waves, half sinewaves, and Gaussian waves.

In the method, the electrical change is attributed to a change in voltage, current, impedance or capacitance.

In the method, both the blood sample and the reagent layer are restrained from undergoing redox reactions in the step (3) when the working electrode and the auxiliary electrode are controlled to have the same voltage.

Preferably, the sample-introducing part of the biosensor has therein a passage ranging in width from 0.5 to 2 mm and in height from 50 to 250 $\mu$m, thereby facilitating the introduction of the blood sample.

In the method, the reagent layer containing the enzyme and the electron transfer mediator is formed on either the working electrode or the auxiliary electrode.

In a preferred embodiment of the method, the reagent layer of the biosensor is formed on both or one of the working electrode and the fluidity-determining electrode, and the two electrodes are arranged such that the steady-sate current time constant is between 0.05 and 8.0, both inclusive.

In a preferred embodiment of the method, the electron transfer mediator is hexaamineruthenim (III) chloride, adapted to facilitate electron transfer from the enzyme to a final electron acceptor, and the reagent layer further comprises a fatty acid or its salt and a quaternary ammonium salt to substantially reduce a hematocrit level-dependent bias.

According to the method of the present invention, a blood sample can be introduced fast and constantly, without being pretreated and accurately analyzed for blood glucose levels within 5 sec.

BRIEF DESCRIPTION OF THE DRAWINGS

The application of the preferred embodiments of the present invention is best understood with reference to the accompanying drawings, in which like reference numerals are used for like and corresponding parts, wherein:

FIG. 1 is an exploded perspective view showing a thin-layer electrochemical cell in accordance with a preferred embodiment of the present invention;

FIG. 2 is an exploded perspective view showing a converse-type biosensor in accordance with a preferred embodiment of the present invention;

FIG. 3a is an exploded perspective view showing a converse-type electrochemical biosensor provided with a sample-introducing part, in which a fluidity-determining electrode is formed on an upper substrate, in accordance with a preferred embodiment of the present invention;

FIG. 3b is an exploded perspective view showing a converse-type electrochemical biosensor provided with a sample-introducing part, in which a fluidity-determining electrode is formed on a lower substrate, in accordance with a preferred embodiment of the present invention;

FIG. 4a is a plan view of a sensor strip-inserted, bidirectional reading device;

FIG. 4b is an internal circuit diagram illustrating the operation process of a biosensor in accordance with a preferred embodiment of the present invention;

FIG. 5 is a cyclic voltammogram of a converse-type biosensor in accordance with a preferred embodiment of the present invention;

FIG. 6 is a chronoamperometric graph showing the comparison of response times between a conserve-type electrode according to a preferred embodiment of the present invention and a flat-type electrode;

FIG. 7 is a chronoamperometric graph showing response time results of a conserve-type electrode according to a preferred embodiment of the present invention at various concentrations of glucose;

FIG. 8 is a graph showing the influence of interfering components on a conserve-type electrode according to a preferred embodiment of the present invention;

FIG. 9 is a graph showing a calibration curve of a converse-type glucose sensor for sensitivity to glucose standard solution;

FIG. 10 is a graph showing dynamic curves, obtained by a chronoamperometric method, of a converse-type glucose sensor for glucose standard solutions; and

FIG. 11 is a graph that illustrates the relationship between the sample fluidity (as a function of time) and the hematocrit level.

DETAILED DESCRIPTION OF THE INVENTION

Reference should now be made to the drawings, in which the same reference numerals are used throughout the different drawings to designate the same or similar components.

The biosensor for measuring blood glucose levels in accordance with the present invention has a thin-layer electrochemical cell in which a working electrode 104 and an auxiliary electrode 105, respectively formed on two flat insulation substrates, are spatially separated from each other by a pressure-adhesive spacer 50-250 $\mu$m thick, symmetrically or asymmetrically face each other, and are electrically connected to each other via a connection line formed along with the working electrode 104 on the same substrate, the connection line having a thick conductive material printed on a portion thereof and three-dimensionally connected to the auxiliary electrode 105 (see: converse-type electrodes: E. K. Bauman et al., Analytical Chemistry, vol 37, p 1378, 1965; K. B. Oldham in "Microelectrodes: Theory and Applications," Kluwer Academic Publishers, 1991).

In the thin spacer, a micro-path on a microliter volume scale is provided for injecting a blood sample in the measurement space defined by the working electrode 104 and the auxiliary electrode 105 and retaining the sample therein. In the thin spacer, a fluidity-determining electrode is placed preferably at such a predetermined distance from the working electrode (or the auxiliary electrode 105) that fluorinated blood with a corpuscle volume of 40% can reach the working electrode (or the auxiliary electrode) along the micro-path 0.5-2 mm wide and 50-250 $\mu$m high within about 600 ms, and more preferably at such a predetermined distance from the working electrode (or the auxiliary electrode 105) that non-fluorinated blood can reach the electrode along the micro-path 0.5-2 mm wide and 50-250 $\mu$m high within 300 ms, and far more preferably within 200 ms.

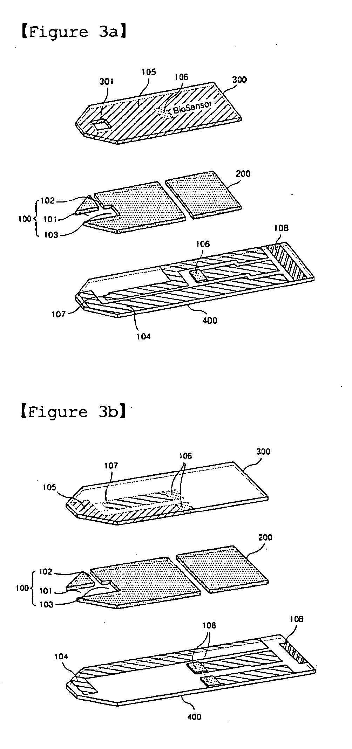

With reference to FIG. 3a, a converse-type biosensor in accordance with an embodiment of the present invention is shown in which a working electrode 104, formed along with a fluidity-determining electrode 107 on the same substrate, faces an auxiliary electrode 105, working as a reference electrode, formed on a separate substrate. FIG. 3b shows a converse-type biosensor in accordance with another embodiment of the present invention, in which an auxiliary electrode, working as a reference electrode, along with a fluidity-determining electrode 107 on the same substrate, faces a working electrode 104.

The biosensor has a structure in which a reagent layer composition solution may be formed on either the working electrode 104 or the auxiliary electrode 105, and preferably on the fluidity-determining electrode 107, as well as either the working electrode 104 or the auxiliary electrode 105.

As shown in FIG. 3a, the electrochemical biosensor in accordance with an embodiment of the present invention comprises a lower substrate 400 upon which are constructed the working electrode and the fluidity-determining electrode, both coated with the reagent layer composition solution, and an electrode connector 106, made from a conductive material, for three-dimensionally connecting the working electrode with the auxiliary electrode; a middle substrate (thin spacer) provided with a cut-out pattern of a sample-introducing part 100 consisting of a sample-introducing.bay 101, an air-discharge channel 102 and an extra void space, wherein the sample-introducing bay is crossed with the air-discharge channel, leaving the void space at the cross; and an upper substrate 300 on which the auxiliary electrode 105, functioning as a reference electrode, is constructed, along with an electrode connector 106, at a position corresponding to the fluidity-determining electrode of the lower substrate, all of said substrates being layered in sequential order in such a way that the structure on the upper substrate faces that on the lower substrate, wherein a reagent layer is preferably formed on the working electrode 104 alone or in combination with the fluidity-determining electrode.

Particularly, the reagent layer containing an enzyme and an electron transfer mediator may be formed only on either the working electrode 104 or the auxiliary electrode while the two electrodes are arranged such that the "steady-state current time constant" (defined as a ratio of the product of a diffusion coefficient of the electron transfer mediator and a steady-state current to the square of the gap between the two electrodes) is between 0.05 and 8.0, both inclusive. Optionally, the reagent layer may further contain a fatty acid and a quaternary ammonium salt.

Through the connection line between the working electrode 104 and the auxiliary electrode 105, a reading device is connected with the biosensor on the same substrate as the working electrode 104. Also, a fluidity-determining electrode 107, positioned on either the insulation substrate of the working electrode 104 or the insulation substrate of the auxiliary electrode 105, functioning to measure the fluidity of a sample, is formed at a suitable distance from the working electrode 104 or the auxiliary electrode 105.

At one end of the spacer for spatially separating the working electrode 104 from the auxiliary electrode 105, a sample-introducing part 100 is formed for introducing a constant amount of a sample into the biosensor therethrough. In detail, the sample-introducing part 100 comprises a sample-introducing bay 101, an air-discharge channel 102 and an extra void space 103. The sample-introducing bay 101 is crossed with the air-discharge channel 102 below the bay end, with the extra void space formed at the cross. The sample-introducing part 100, thus formed in a r shape, allows a blood sample to be introduced from the fore-end of the biosensor strip accurately and conveniently. Notably, the sample-introducing bay 101 communicates with the air discharge-channel 102 in a roughly perpendicular manner slightly below the end of the bay-shaped channel, forming the extra void space 103 behind the point of communication. The term "crossed with" as used herein means that the sample-introducing bay 101 and the air-discharge channel 102 are not linearly arranged, but intersect each other at a predetermined point. During measurement, the extra void space 103 helps hold a constant and accurate volume of the blood sample within the bay while discharging the excess sample through the air-discharge channel 102. Also, the extra void space 103 serves to prevent the formation of air bubbles, which may often occur at the point of communication between the sample-introducing bay 101 and the air-discharge channel 102. The formation of air bubbles may result in inaccurate measurements. To ensure a exact sampling with no bubble formation, hydrophilic treatment of the sample-introducing bay 101 including the extra void space 103 is desired.

The ratio between the widths of the air-discharge channel 102 and the sample-introducing bay 101 is no more than 1:2, and preferably in the range from 1:5 to 1:2. A ratio below 1:2 ensures the containment of an exact amount of a sample in sample-introducing bay 101, and allows the sample to proceed to the air-discharge channel 102 at a high speed.

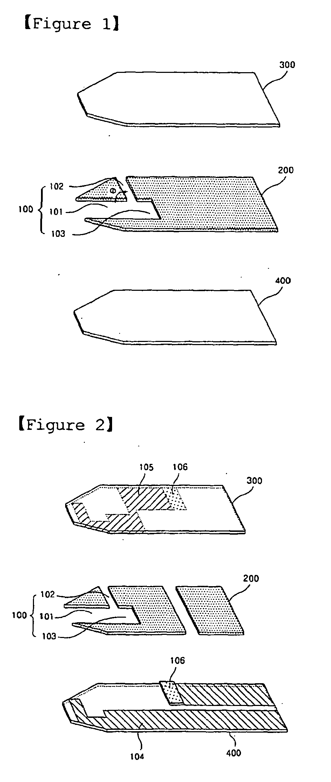

In Fig. 1, the angle of communication ($\phi$) between the sample-introducing bay 101 and the air-discharge channel 102 is shown to be 90°. But the angle may be varied within a range from about 45° to 135°, preferably within a range from about 60° to 105°, and most preferably within a range from about 75° to 105°.

The sample-introducing part 100 preferably has a capacity for retaining 0.1-3.0 $\mu\ell$ of a sample. More preferably, the capacity is in the range from 0.1 to 1.0 $\mu\ell$, and most preferably in the range from 0.3 to 0.7 $\mu\ell$. A sample volume less than 0.1 $\mu\ell$ is too small to give an accurate measurement within the error range of the biosensor. On the other hand, a sample volume greater than 3.0 $\mu\ell$ is excessive for sampling. The sample-introducing bay 101 is utilized as a place on which the fluidity-determining electrode 107 is positioned.

In cooperation with the sample-introducing part 100 and the path, the fluidity-determining electrode 107 acts to measure the fluidity of whole blood samples. Since hematocrits change the fluidity and electrical conductivity of whole blood, sampling times through the Γ-shaped capillary passage suggested in the present invention vary proportionally with the level of hematocrits in whole blood samples. The change in the fluidity of blood samples, which is detected by the fluidity-determining electrode 107, may be used to correct the hematocrit level-dependent bias in the blood glucose measurements. Meanwhile, the fluidity of blood is greatly changed in an old strip or in the case that the reagent layer formed on the working electrode 104 has an unsuitable composition. Hence, the flow rate of blood detected by the fluidity-determining electrode 107 can be used to estimate the time when the biosensor was manufactured or to correct any errors made during the manufacture thereof.

While passing, the working electrode 104 and the fluidity-determining electrode 107, in sequential order or in reverse order, from the inlet of the thin-layer electrochemical cell, a blood sample twice causes an electrical change in voltage, current, impedance or capacitance so as to provide information about the time period of the passage of the sample through the passage. Therefore, taking advantage of the flow speed of a sample on the micro-passage of the thin-layer electrochemical cell, the biosensor provides the function of accurately determining the level of a substrate in the sample or of informing of its manufacture year or errors.

Additionally, the biosensor of the present invention may be provided with a viewing window 301 on the upper substrate 300, which is located above a portion of the fluidity-determining electrode on the lower substrate. The viewing window 301 makes it possible to visually determine whether a sample is filled or not.

In the electrode structure described above, the current reaches a steady state within a few seconds due to the cycling effect of the redox reactions formed by an enzyme, a substrate contained in the sample, and an electron transfer mediator. In this regard, the reagent layer formed on either the working electrode or the auxiliary electrode has to be readily dissolved by the sample introduced through the sample channel.

When used in a proper concentration, hexaamineruthenium (III) chloride can transfer electrons tens of times faster than can Fe-based electron transfer mediators. In accordance with the present invention, the reagent layer is made from a composition comprising hexaamineruthenium (III) chloride, a fatty acid, a quaternary ammonium salt, and an auxiliary enzyme dispersant, which is readily dissolved in blood and able to substantially reduce the hematocrit level-dependent bias.

Therefore, the biosensor of the present invention comprises a reagent layer composition solution capable of reducing the measurement error attributed to the hematocrit level of blood. That is, the reagent layer composition solution comprises an enzyme, an electron transfer mediator, a water-soluble polymer, a fatty acid and a quaternary ammonium salt.

In the biosensor, the reagent layer composition solution functions to outstandingly reduce the effect of hematocrits in addition to excluding the influence of interfering components such as ascorbic acid, acetaminophen and uric acid.

As seen in Reaction Formula I, the enzyme reacts with a metabolite of interest, with the cofactor being reduced. Then, the reduced cofactor transfers electrons to the electron transfer mediator, thereby quantitatively analyzing the metabolite of interest.

Herein, it should be noted that the present invention, although described for biosensors for the analysis of blood glucose levels, can introduce appropriate enzymes and electron transfer mediators to the electrode system so that a variety of samples, including bio-materials, such as metabolites, e.g., cholesterol, lactate, creatinine, proteins, hydrogen peroxide, alcohols, amino acids, and enzymes, e.g., GPT (glutamate pyruvate transaminase) and GOT (glutamate oxaloacetate transaminase), environmental materials, agricultural and industrial materials, and food materials, can be quantitatively analyzed. That is, versatile metabolites can be analyzed for their levels once suitable enzymes are selected in concert with the electron transfer mediator. For instance, like glucose oxidase used for the quantitative analysis of glucose level, lactate oxidase can be applied to lactate, cholesterol oxidase to cholesterol, glutamate oxidase to glutamate, horseradish peroxidase to hydrogen peroxide, and alcohol oxidase to alcohol. Preferably, the enzyme suitable for use in the present invention is selected from the group consisting of GOx (glucose oxidase), GDH (glucose dehydrogenase), cholesterol oxidase, cholesterol esterifying enzyme, lactate oxidase, ascorbic acid oxidase, alcohol oxidase, alcohol dehydrogenase, bilirubin oxidase, glucose dehydrogenase. In examples of the present invention, the biosensor employed glucose oxidase or glucose dehydrogenase to analyze blood glucose levels.

When reacting with the reduced cofactor of the enzyme, the electron transfer mediator is reduced. The diffusion of the reduced electron transfer mediator to the surface of the electrodes causes the application of an anodic potential to the working electrode, generating electricity.

As an electron transfer mediator, ferrocene or its derivatives, quinone or its derivatives, organic conducting salts, or viologen may be used. Preferably, the electron transfer mediator is a mixed-valence compound able to form a redox couple, including hexaamineruthenium (III) chloride, potassium ferricyanide, potassium ferrocyanide, DMF (dimethylferrocene), ferricinium, FCOOH (ferocene monocarboxylic acid), TCNQ (7,7,8,8-tetracyanoquinodimethane), TTF (tetrathiafulvalene), Nc (nickelocene), NMA+ (N-methylacidinium), TTT (tetrathiatetracene), NMP+ (N-methylphenazinium), hydroquinone, MBTHDMAB (3-dimethylaminobenzoic acid), 3-methyl-2-benzothiozolinone hydrazone, 2-methoxy-4-allylphenol, AAP (4-aminoantipyrin), dimethylaniline, 4-aminoantipyrene, 4-methoxynaphthol, TMB (3,3',5,5'-tetramethylbenzidine), 2,2-azino-di-[3-ethylbenzthiazoline sulfonate], o-dianisidine, o-toluidine, 2,4-dichloro phenol, 4-aminophenazone, benzidine, and Prussian blue.

Of these, hexaamineruthenium (III) chloride is preferred because its formal potential is low enough to minimize the influence of various interfering components, such as ascorbic acid, acetaminophen and uric acid.

The water-soluble polymer that helps the reaction of the enzyme is contained in an amount from 0.1 to 10 wt% based on the total weight of the reagent layer composition solution in a solid state. Examples of the water-soluble polymer suitable for use in the present invention include PVP (polyvinyl pyrrolidone), PVA (polyvinyl alcohol), perfluoro sulfonate, HEC (hydroxyethyl cellulose), HPC (hydroxypropyl cellulose), CMC (carboxy methyl cellulose), cellulose acetate, and polyamides, with preference for PVP and HPC.

Playing an important role in reducing the hematocrit level-dependent bias, both a fatty acid and a quaternary ammonium salt are contained in the reagent layer composition solution of the present invention.

When used, a fatty acid tends to shorten the linear dynamic range of a biosensor, especially in the high-concentration region, in addition to greatly helping reducing the hematocrit level-dependent bias.

Before being added to the solution, a fatty acid is dissolved in water or a water-miscible solvent. The fatty acid is used in an amount from 0.1 to 20 wt% of all solid components of the solution. Suitable is a fatty acid containing 4-20 carbon atoms or its salt. A saturated fatty acid with an alkyl chain of 6-12 carbons or its salt is preferred. Examples of the fatty acid suitable for use in the present invention include caproic acid, heptanoic acid, caprylic acid, nonanoic acid, capric acid, undecanoic acid, and lauric acid, tridecanoic acid, myristic acid, pentadecanoic acid, palmitic acid, heptadecanoic acid, stearic acid, nonadecanoic acid, and arachidic acid.

In cooperation with the fatty acid, a quaternary ammonium salt can further reduce the hematocrit level-dependent bias.

A suitable quaternary ammonium salt may be exemplified by halide compounds of dodecyltrimethylammonium, myristyltrimethylammonium, cetyltrimethylammonium, octadecyltrimethylammonium, tetrahexylammonium, etc. The quaternary ammonium salt is used in an amount from 0.1 to 30 wt% of all components of the reagent layer composition solution.

The reagent layer is formed on the working electrode simply by dispensing a drop of the reagent layer composition solution with the aid of a dispenser. The drop of the reagent layer composition is preferably about 300-500 nl or more preferably 200 nl or less.

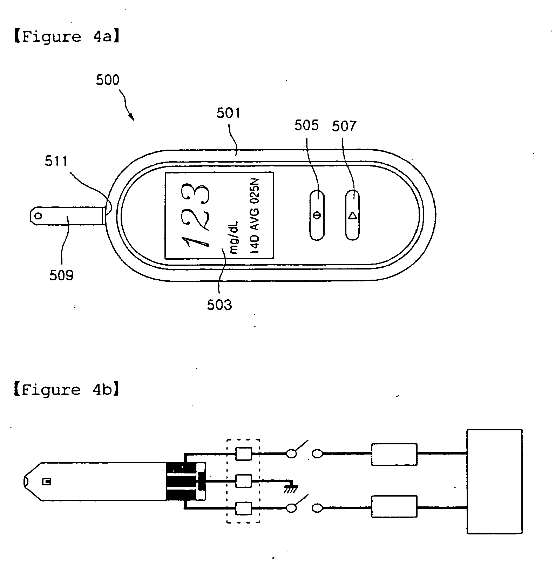

A description will now be given of a method of measuring blood glucose levels using the biosensor of the present invention. The method is conducted according to the following steps, using a reading device to which a sensor strip 509 is applied as shown in FIG. 4a. The operational concept of the biosensor is schematically illustrated in the circuit diagram of FIG. 4b.

In Step 1, a blood sample taken from the forearm is introduced to the reading device 500 into which the sensor strip (thin-layer electrochemical cell) 509 is inserted.

A sample volume suitable for quantitative assay with minimal pain to the patient is in the range from 0.1 to 0.7 $\mu$l. The biosensor of the present invention not only requires no treatment of the blood sample for analysis, but also enables accurate and rapid measurement of blood glucose levels. This is partly attributed to the passage ranging in width from 0.5 to 2 mm and in height from 50 to 250 $\mu$m, which is formed in the sample-introducing part 100 of the biosensor so as to facilitate the introduction of blood samples by way of capillary action.

In Step 2, constant potential differences are respectively given between the working electrode 104 and the auxiliary electrode 105 and between the fluidity-determining electrode 107 and the auxiliary electrode 105.

As soon as the biosensor detects the insertion of the biosensor strip into the reading device (Step 1), predetermined constant potentials are applied between the working electrode 104 and the auxiliary electrode 105 and between the fluidity-determining electrode 107 and the auxiliary electrode 105. The potential applied to the working electrode 104 is independent of that applied to the auxiliary electrode 107, with the total circuit forming an open circuit. An electrical change according to sample introduction becomes a potential difference in an open circuit state and the potential difference signal is used as a starting signal in the course of the measurement of the biosensor.

The reagent layer containing an enzyme and an electron transfer mediator is formed on either the working electrode 104 or the auxiliary electrode 105 and these electrodes are arranged at such a gap that the steady-state current time constant is in a range from 0.05 to 8.0. For the measurement of blood glucose levels, GOx (glucose oxidase) or GDH (glucose dehydrogenase) is employed in the biosensor. Also, hexaamineruthenium (III) chloride is selected as the electron transfer mediator. For the reason mentioned above, the reagent layer may contain a fatty acid and a quaternary ammonium salt.

The reagent layer composition solution is dispensed to only the working electrode 104 or both the working electrode 104 and the fluidity-determining electrode 107 and these electrodes are preferably arranged at such a gap that the steady-state current time constant is in the range from 0.05 to 8.0.

In Step 3, the introduction of a blood sample causes a primary electrical change between the working electrode 104 and the auxiliary electrode 105 and the electrodes are controlled to have the same voltage. In order to achieve the same voltage, a current is allowed to flow therebetween while the redox reaction between the sample and the reagent layer is restrained within a few seconds. The restraint of the redox reaction is continuously kept for 0.001 to 3 sec.

The insertion of the strip into the reading device does not lead to the connection of the total circuit. When a blood sample is introduced through the sample-introducing part 100, the flow of a primary instant current is sensed and the measurement of the flow time period is initiated. The sample introduced to the passage mouth of the strip contains electrolytes therein, thus serving to switch the circuit on to flow a current between the working electrode 104 and the auxiliary electrode 105 via the electrode connector 106 therebetween. Making the voltage the same between the working electrode 104 and the auxiliary electrode 105, the current restrains the redox reaction of the sample for the time period at which the sample is mixed with the reagent layer, preferably for 3 sec and more preferably for 2 sec or less. During this step, the circuit remains closed.

In Step 2, when contacting the blood sample, the fluidity-determining electrode 107 senses its fluidity to cause a secondary electrical change, which leads to a control into the same voltage between the auxiliary electrode 105 and the fluidity-determining electrode 107. Thus, information about the time difference between the primary and the secondary electrical change is detected.

As soon as a sample contacts the extra void space 103, a second instant current is sensed and a time gap between the first and the second instant current is recorded. In detail, when being introduced to the mouth of the thin-layer electrochemical cell, a sample passes the working electrode 104 and the fluidity-determining electrode 107 in sequential order or in reverse order, causing an electrical change in voltage, current, impedance or capacitance twice, which leads to the information about the flow period of time of the sample through the passage.

The fluidity-determining electrode 107 is positioned preferably at such a predetermined distance from the working electrode 104 that fluorinated blood with a corpuscle volume of 40% can reach the working electrode along the passage 0.5-2 mm wide and 50-250 $\mu$m high, within about 600 ms and that non-fluorinated blood can reach the electrode along the passage 0.5-2 mm wide and 50-250 $\mu$m high, within 300 ms and more preferably within 200 ms.

Together with the fluidity-determining electrode 107, the sample-introducing part 100 and the passage form a structure suitable for measure the fluidity of whole blood samples. The fluidity of a sample is determined as a function of the speed at which the sample fills the space between the first contact point of the electrode near the mouth of the sample-introducing part 100 and the fluidity-determining electrode 107 which is located at either the void 103 or the air-discharge channel 102.

The passage formed in the thin spacer 50-250 $\mu$m thick comprises a linear sample-introducing bay 101 in which a constant volume of a sample is held and an air-discharge channel 102 which helps the capillary action of the sample-introducing part 100. Since hematocrits change the fluidity and electrical conductivity of whole blood, the time period of the passage of blood through the $\Gamma$-shaped capillary channel of the biosensor strip varies proportionally with the level of hematocrits in whole blood samples. Detected by the fluidity-determining electrode 107, such variances in the fluidity of blood samples may be used to correct the hematocrit level-dependent bias in the blood glucose measurements. Also, the fluidity of blood is greatly changed in an old strip or in the case that the reagent layer formed on the working electrode 104 has an inappropriate composition. Hence, the flow rate of blood detected by the fluidity-determining electrode 107 can be used to estimate the time when the biosensor was manufactured or to correct the error made during the manufacture thereof.

A fitting equation between sample filling time X and hematocrit level Y is given as follows:

$$\text{Mathematical Formula I}$$

$$Y = -72.23 + 0.58691X - 0.00084073\ X^2 - 1.1211 \times 10^{-6}\ X^3 + 5.7521 \times 10^{-9}\ X^4 - 9.1172 \times 10^{-12}\ X^5$$

The fluidity-determining electrode 107 discerns the abnormal fluidity of blood samples, which may result from too high or low a hematocrit of blood samples or the introduction of a bubbled blood sample. In such abnormal cases, warning messages or error codes according to an installed program appear on the reading device.

In Step 5, when the blood sample is sufficiently mixed with the reagent layer on the working electrode 104, a predetermined voltage is applied between the working electrode 104 and the auxiliary electrode 105 so as to cause the cycling reactions in the converse-type thin-layer electrochemical cell, followed by reading the steady-state current thus obtained. The steady state is reacheds within seconds, e.g., 2-10 sec. Compared to conventional flat-type electrodes, the converse-type electrodes of the present invention have advantages in terms of fast response time and high steady-state current. Although depending on the reaction rate and electron transfer rate of the electron transfer mediator used, the converse-type electrodes provide steady-state currents within a short time.

In Step 6, taking advantage of the time information obtained in Step 4 and the steady-state current obtained in Step 5, the biosensor determines the level of the substrate in the sample. In accordance with the present invention, the measurement of blood glucose levels is performed within 5, preferably within 4 sec, and more preferably within 3 sec.

As described above, the measurement of blood glucose levels in accordance with the present invention is achieved by subjecting the analyte of interest taken from blood to continuous cycles of redox reactions with the aid of an appropriate enzyme and an electron transfer mediator and then determining the quantity of electrons transferred during the reactions to quantitatively analyze the substrate. Further, the information about the speed at which the sample flows through the micro passage of the thin-layer electrochemical cell helps to more accurately determine the quantity of the substrate and obtain information about the manufacture or storage state of the biosensor.

Alternatively, Steps 1 to 6 may be modified as follows.

(1) A blood sample is introduced into the strip-inserted reading device (Step 1).

(2) Alternating voltages with high constant frequencies are applied between the working electrode 104 and the auxiliary electrode 105 and between the fluidity-determining electrode 107 and the auxiliary electrode 105. The voltages applied to the working electrode 104 and the fluidity-determining electrode 107 are independent, with the total circuit forming an open circuit (Step 2). While alternating voltages are applied between the working electrode 104 and the auxiliary electrode 105 and between the fluidity-determining electrode 107 and the auxiliary electrode 105, an electrical change according to sample introduction appears in a capacitance, so that it can be used as a starting signal in the course of the measurement of the biosensor.

(3) The sample introduced to the mouth of the passage in the strip causes a primary change in the capacitance between the working electrode 104 and the auxiliary electrode 105. Allowing the application of the same voltage to the working electrode and the auxiliary electrode 105, this capacitance change causes the redox reaction to halt for several seconds, during which the sample is mixed with the reagent layer, preferably for 3 sec or less and more preferably for 2 sec or less. During this process, the circuit is maintained to be closed (Step 3).

(4) When the sample contacts the fluidity-determining electrode 107, a secondary capacitance change occurs and allows the auxiliary electrode 105 and the fluidity-determining electrode 107 to have the same voltage, providing the information about the time difference from the change detected by the working electrode 104 (Step 4).

(5) When the blood sample is sufficiently mixed with the reagent layer on the working electrode 104, a predetermined constant potential difference is applied between the working electrode 104 and the auxiliary electrode 105 so as to initiate the cycling reactions characteristic of the converse-type thin-layer electrochemical cell. Within several seconds, and preferably within 2 seconds, the current flowing between the electrodes reaches a steady state and is read (Step 5).

(6) The level of the substrate in the tested sample is determined on the basis of the time information obtained in Step 4 and the steady-state current read in Step 5. The entire process, from the introduction of a sample to the determination of substrate level, is completely conducted within seconds, preferably within 4 seconds, and more preferably within 3 seconds.

Another embodiment of the method may be achieved as follows.

(1) When a blood sample is introduced into the strip-inserted reading device, a high impedance input circuits between the working electrode 104 and the auxiliary electrode 105 and between the fluidity-determining electrode 107 and the auxiliary electrode 105 are activated in the reading device (Step 1).

(2) Because the sample introduced to the mouth of the passage in the strip contains electrolytes, a primary potential difference is formed at the interface between the electrode and the sample (Step 2).

(3) This change is detected to allow the application of the same voltage to the working electrode, causing the redox reaction to halt for several seconds, during which the sample is mixed with the reagent layer, preferably for 3 sec or less, and more preferably for 2 sec or less. During this process, the circuit is kept to be closed (Step 3).

(4) When the sample contacts the fluidity-determining electrode 107, a secondary voltage change occurs and allows the auxiliary electrode 105 and the fluidity-determining electrode 107 to have the same voltage, providing the information about the time difference from the change detected by the working electrode 104 (Step 4).

(5) When the blood sample is sufficiently mixed with the reagent layer on the working electrode 104, a predetermined constant potential difference is applied between the working electrode 104 and the auxiliary electrode 105 so as to initiate the cycling reactions characteristic of the converse-type thin-layer electrochemical cell. Within seconds, and preferably within 2 seconds, the current flowing between the electrodes reaches a steady state and is read (Step 5).

(6) The level of the substrate in the tested sample is determined on the basis of the time information obtained in Step 4 and the steady-state current read in Step 5. The entire process, from the introduction of a sample to the determination of substrate level, is completely conducted within seconds, preferably within 4 seconds, and more preferably within 3 seconds.

In addition, direct currents, low- or high-frequency alternating currents, high impedances, or various types of pulses, such as square waves, pyramidal waves, half sinewaves, or Gaussian waves, may be applied between the working electrode 104 and the auxiliary electrode 105 and between the fluidity-determining electrode 107 and the auxiliary electrode 105 in order to quantitatively analyze the substrate of interest. In these cases, however, the determination of sampling time based on the chemical change occurring upon sample introduction is independent of the time it takes to apply and measure the electrical signals between the working electrode 104 and the reference and auxiliary electrode 105, but is used to correct the electrochemical change caused by the chemical reactions, along with the information about the traveling time of the sample between the working electrode and the fluidity-determining electrode. This is performed with pre-installed software.

The measuring method according to the present invention can substantially reduce the hematocrit level-dependent bias, taking advantage of the information on sample fluidity in addition to decreasing the possibility of obtaining false information by excluding the blood sample of abnormally high or low fluidity. Further, the biosensor itself can detect, the change in blood sampling speed caused by the aging thereof, thereby providing the information about quality control upon manufacture. Moreover, the measuring method of the present invention is advantageous in that a blood sample can be introduced fast and constantly, without being pretreated and accurately analyzed for blood glucose levels within 5 sec.

The biosensors provided with the sample-introducing part 100 enjoy the following advantages:

(1) The air-pocket phenomenon, which may occur at the point of communication between the sample-introducing bay and the air-discharge channel upon the rapid introduction of the sample by way of capillary action, is eliminated by the extra void space provided behind the point of communication.

(2) As the sample-introducing part 100 is well enclosed by the narrow mouth and the air-discharge channel and the entire passage is covered with the upper substrate 300, there is almost no possibility that the biosensors of the present invention leak the introduced sample to stain the hand of the user with the sample. Also, the biosensors can maintain a consistent sample concentration therein with minimal evaporation, thus improving analytical reproducibility.

(3) The biosensors provided with the sample-introducing part 100, in which the sample-introducing bay 101 communicates with the air-discharge channel 102 in a roughly perpendicular manner, are capable of rapidly introducing a predetermined amount of sampled blood thereinto and increasing accuracy and reproducibility.

(4) Finally, the biosensors of the present invention are more convenient for blood sampling because the sample-introducing part 100, adapted at the tip, can be readily brought into contact with body parts.

A better understanding of the present invention may be given with the following examples which are set forth to illustrate, but are not to be construed to limit the present invention.

EXAMPLE 1: Preparation of Reagent Layer Composition Solution Without Fatty Acid

A mixture containing 30 mg of hexaamineruthenium (III) chloride (41.6 wt%), 1 mg of carboxymethylcellulose (1.4 wt%), 1 mg of Triton X-100 (1.4 wt%), and 40 mg of glucose oxidase (55.6 wt%) was dissolved in 1 ml of PBS buffer (pH 6.4), followed by filtering off undissolved particles. The reagent solution thus obtained was placed in the syringe of a pneumatic dispenser (EFD XL100).

EXAMPLE 2: Preparation of Reagent Layer Composition Solution With Fatty Acid

A mixture containing 30 mg of hexaamineruthenium (III) chloride (32.6 wt%), 1 mg of carboxymethylcellulose (0.8 wt%), 5 mg of polyvinyl pyrrolidone (4 wt%), 1 mg of Triton X-100 (0.8 wt%), 20 mg of lauric acid (15.7 wt%), 30 mg of myristyltrimethylammonium bromide (23.6 wt%), and 40 mg of glucose oxidase (31.5 wt%) was dissolved in 1 ml of PBS buffer (pH 6.4), followed by filtering off undissolved particles. The reagent solution thus obtained was placed in the syringe of a pneumatic dispenser (EFD XL100).

EXAMPLE 3

In this example, a method of measuring blood glucose levels is described. The auxiliary electrode 105 shown in FIGS. 2 and 3b was used as a reference electrode in the thin-layer electrochemical cell-type biosensor. A thin-layer electrochemical cell for the measurement of blood glucose levels was fabricated as follows.

As shown in FIGS. 2, 3a and 3b, a working electrode 104, and an electrode connector 106, which is thick enough to three-dimensionally connect with an auxiliary electrode, were screen-printed with conductive carbon paste, and then cured at 140°C for five minutes. Next, a circuit connector was screen-printed with a silver paste on one end of the electrode connector 106 to the thickness of the middle substrate 200. Likewise, a reference (auxiliary) electrode 105 was screen-printed with carbon paste on the upper substrate 300 and cured in the same condition as in the electrode of the lower substrate 400. Finally, a silver paste was screen-printed at the end of the reference electrode 105 to afford a circuit connector.

The middle substrate 200, in which the sample introducing bay 101, the air-discharge channel 102, and the extra void space 103 are arranged in such a way that the end of the fluidity-determining electrode 107 is positioned in the extra void space, was prepared by pressing a double-sided polyester tape. In this connection, the structure of the middle substrate was designed such that the width ratio of the air-discharge channel 102 to the sample introducing bay 101 was 2:1 and the sample introducing part 100 had a capacity of 0.5 $\mu$l.

In order to assemble the substrates in the biosensor, as shown in FIGS. 2, 3a and 3b, the middle substrate 200 was pressed against the electrode-printed lower substrate 400, followed by applying the reagent layer composition solution prepared in Example 1 or 2 to the working electrode 104 exposed through the sample passage. After the working electrode was dried at 45°C for 30 min, the upper substrate 300 was pressed against the middle substrate 200 in such a way that the circuit connectors formed on the respective substrates were brought into contact with each other, thereby fabricating a converse-type biosensor. FIGS. 3a and 3b show biosensors having the fluidity-determining electrode formed on the lower substrate and the upper substrate, respectively.

In order to measure blood glucose levels using the fabricated sensor, 0.5 $\mu$l of blood was taken from the forearm of a patient with a sensor strip. As soon as the strip was inserted into a reading device, it started to operate. The insertion of the strip into the reading device caused a potential difference of 200 mV to be applied to the working electrode 104 and the auxiliary electrode 105 within the cell.

In more detail, the strip-inserted reading device operated as follows.

When a blood sample was introduced by combining the sensor strip with the reading device 500, (Step 1), a predetermined potential difference was given between the working electrode 104 and the auxiliary electrode 105 and between the fluidity-determining electrode 107 and the auxiliary electrode 105 (Step 2). The sample introduced to the mouth of the passage in the strip caused a primary change in electrical signal between the working electrode 104 and the auxiliary electrode 105, which led to allowing the application of the same voltage to the working electrode and the auxiliary electrode 105 (Step 3). Then, when sensing the flow of the sample, the fluidity-determining electrode 107 generated a secondary electrical change, which also resulted in allowing the auxiliary electrode 105 and the fluidity-determining electrode 107 to have the same voltage, providing the information about the time difference from the change detected by the working electrode 104 (Step 4). When the blood sample was sufficiently mixed with the reagent layer on the working electrode 104, 200 mV was applied between the working electrode 104 and the auxiliary electrode 105 so as to initiate the cycling reactions in the converse-type thin-layer electrochemical cell, followed by reading the ready current thus obtained (Step 5). Finally, the level of the substrate in the sample was determined on the basis of the time information obtained in Step 4 and the steady-state current read in Step 5 (Step 6).

Through a series of these steps, the biosensors of the present invention could measure blood glucose levels readily, rapidly, and accurately.

EXPERIMENTAL EXAMPLE 1: Cyclic Voltammogram Depending on Scanning Speed of Converse-Type Glucose Sensor

Using the converse-type electrodes fabricated in Example 3, cyclic voltammogram was examined according to scanning speed.

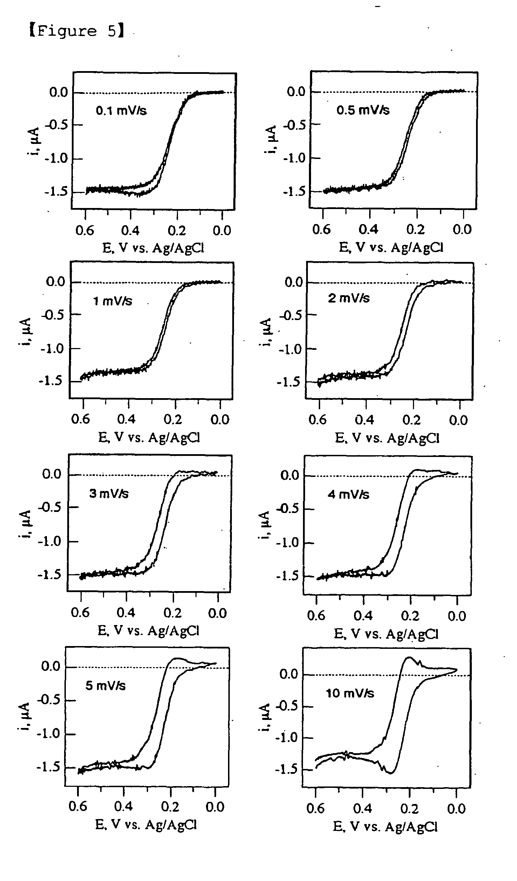

A test was performed in a 1M KCl solution containing 0.05 mM[Fe(CN)$_6$/[Fe(CN))$_6$]$^{4-}$. At scanning speeds of 3 mV/s or less, the electrodes of the present invention exhibited S-curves, which are characteristic of steady-state currents of microelectrodes. Under a limiting condition, the concentration at surfaces of the two electrodes became zero while the current therebetween was independent of the potential and also the limiting current was independent of the potential. As the scanning speed increased, the hysteresis between a reversible and an irreversible stage also increased. When v became as large as or larger than 20 mV/s, CV in the converse-type electrodes of the present invention exhibited the same signal shape as in flat-type electrodes, but not an S signal shape. The results are shown in FIG. 5. Peak separation occurred at a CV of about 55 mV, representing a high reversible electron transfer process between the electrode and [Fe(CN)$_6$]$^{3-/4-}$.

Taken together, these results are similar to those of the study on random assembled microelectrodes due to the change of hemispherical diffusion into lateral diffusion on a linear sweep voltammeter, suggesting that the carbon electrodes of the present invention have properties similar to those of the microelectrodes formed of carbon particles of random sizes.

EXPERIMENTAL EXAMPLE 2: Comparison of Chronoamperometric Response Time Between Converse-Type Glucose Sensor and Flat-Type Sensor

The chronoamperometric response time of the converse-type electrodes fabricated in Example 3 were compared with that of flat-type electrodes, which are arranged on one insulation substrate, as follows.

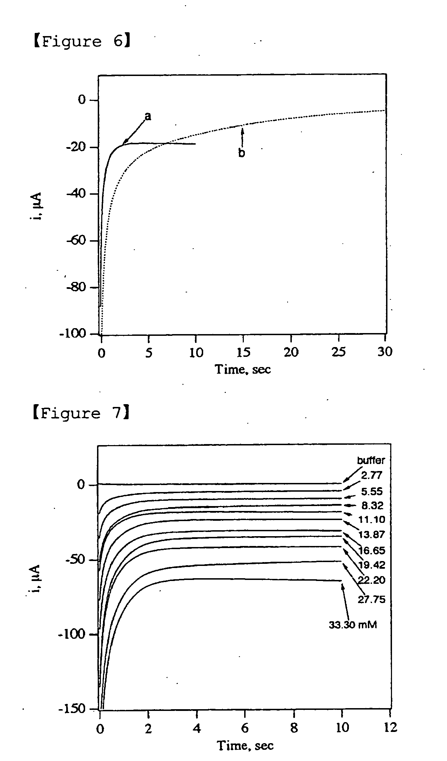

As compared with flat-type electrodes, the converse-type electrodes exhibited higher response speeds to sample and larger steady-state currents. The results are given in FIG. 6. If the reaction rate and electron transfer rate of the electron transfer mediator used was appropriate, the converse-type electrodes provided steady-state currents within a very short time. In this connection, the steady-state time constant ($t^*=Dt/d^2$) was introduced to determine the condition of the steady-state currents varying with the arrangement of electrodes and the composition of the reagent layer.

In the response curve of a biosensor fabricated according to an embodiment of the present invention, the time needed to reach a steady-state current was about 2 sec, and the diffusion coefficient of the electron transfer mediator hexaamineruthenium (III) chloride was $1.8\times10^{-5}$ cm$^2$/s, with a 100-$\mu$m gap between electrodes, so that $t^*=0.36$.

A previous research result (J. F. Cassidy et al., Analyst, 118, 415 (1993)) teaches that a steady-state current is obtained when $t^*>0.01$. The biosensors proposed by the present invention satisfy the condition of $0.05 \leq t^* \leq 8$.

FIG. 7 shows chronoamperometric response time results as the concentration of glucose increases from 2.77 to 33.3 mM. The response time increases with glucose concentration. However, as shown in the graph, all cases tested allowed steady-state currents to be determined within 2 sec. Such fast and complete steady-state current responses make it possible to process data at an improved rate and enhance the analytical implement of the electrodes.

EXPERIMENTAL EXAMPLE 3: Influence of Interfering Components on a Converse-Type Glucose Sensor

The influence of interfering components, such as ascorbic acid, acetaminophen and uric acid, on a converse type glucose sensor having a 0.5 $\mu$l sample introducing part 100, fabricated as depicted in example 3, was measured.

Particularly, respective response currents to (a) a solution of 177 mg/dL of glucose in phosphate buffer (pH 7.4) (b) a solution of 177 mg/dL of glucose + 660 $\mu$M of acetaminophen in PBS buffer, (c) a solution of 177 mg/dL of glucose + 570 $\mu$M of ascorbic acid in PBS buffer, and (d) a solution of 177 mg/dL of glucose + 916 $\mu$M of uric acid in PBS buffer were measured.

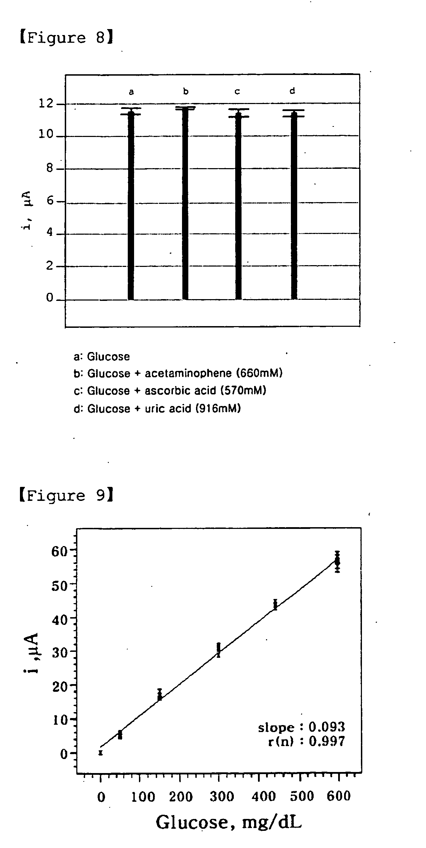

The currents were determined by reading chronoamperometric responses 5 seconds after the application of +0.2 V potential to the working electrode 104 (vs. the reference electrode). The results are shown in FIG. 8.

As seen in FIG. 8, there is no notable difference between the results from PBS buffer solutions containing 177 mg/dL of glucose alone (line a) and in combination with 660 $\mu$M of acetaminophen (line b), 570 $\mu$M of ascorbic acid (line c), or 916 $\mu$M of uric acid (line d). Consequently, these data show that the sensors are insignificantly affected by the presence of interfering materials at an applied potential of +0.2 V.

EXPERIMENTAL EXAMPLE 2: Calibration Curve of Converse-type Glucose Sensor to Glucose Standard Solution

The converse-type glucose sensor prepared in Example 3 was assayed for sensitivity with glucose standard solutions.

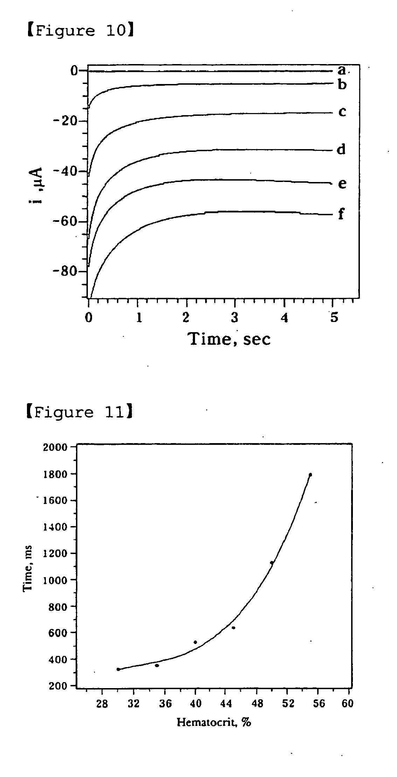

In detail, current values were measured ten times at each glucose concentration 0, 50, 150, 300, 450 or 600 mg/dL in the presence of an electrical field for the applied potential of 0.2 V with respect to the reference electrode. The amount of samples applied to the sample introducing part was 0.5 $\mu$l and the filling time was no more than 200 ms. The measurements were performed 2 sec after the introduction of the sample by applying 0.2 V for 3 sec, and the current values were read in 5 sec. The results are depicted in FIG. 10.

FIG. 10 shows dynamic response curves obtained at glucose concentrations of 0mg/dL (curve a), 50mg/dL (curve b), 150mg/dL (curve c), 300mg/dL (curve d), 450 mg/dL (curve e), and 600mg/dL (curve f).

As is apparent from the curves, the biosensor of the present invention can reach steady-state currents, assuring the rapid and accurate measurements.

In the biosensor of the present invention, the slope was 0.093 [$\mu$A/(mg/dL)] and the correlation coefficient was 0.997. From these results, the electrochemical biosensor was proven to have excellent linear sensitivity (FIG. 9).

EXPERIMENTAL EXAMPLE 5: Blood Fluidity Measurement and Hematocrit Bias Correction

Using a converse-type glucose sensor provided with a fluidity-determining electrode, fabricated as in Example 3, assays were performed for blood fluidity measurement and hematocrit bias correction.

200 mV of potential was applied to the working electrode 104 and the fluidity-determining electrode 107 (vs. the reference electrode 105). When blood samples were introduced through the sample-introducing bay 101, a first sudden change in current was detected, and the time measurement begins. As soon as the sample reaches the void 103, a second surge of current was detected and the time interval between the first and second surge of current was recorded. The relationship between the sample introducing time and hematocrit level is shown in FIG. 11. The experiment was performed with the sodium fluoride-treated whole blood containing 180 mg/dL of glucose and varying hematocrit levels.

The fitting equation was obtained by the above result.

[Mathematical Formula  1]

$$Y = -72.23 + 0.58691X - 0.00084073\ X^2 - 1.1211 \times 10^{-6}\ X^3 + 5.7521 \times 10^{-9}\ X^4 - 9.1172 \times 10^{-12}\ X^5$$

(where Y is the estimated hematocrit level from the sample filling time X measured with the fluidity-determining electrode)

Table 1 shows the level of hematocrit estimated from the speed of sample-filling time.

Table 1. Hematocrit levels estimated from the sample-filling time of the biosensor prepared in Example 3.

| Hematocrit (%) Prepared sample | Speed (ms) | Estimated Hematocrit (%) |
| --- | --- | --- |
| 30% | 326 | 30.3% |
| 35% | 352 | 32.8% |
| 40% | 530 | 41.8% |
| 45% | 634 | 44.0% |
| 50% | 1129 | 50.1% |
| 55% | 1791 | 54.7% |

In a separate experiment, calibration curves were obtained with the whole blood at various hematocrit levels and the relationship between the hematocrit level and the response slopes was formulated in Table 2, below.

Table 2. Calibration curves at different hematocrit levels.

| Hematocrit | Equation (y = current $\mu$A; x = glucose) |
| --- | --- |
| 30% | y = 0.035934 x - 1.7228 |
| 35% | y = 0.030559 x - 1.31815 |
| 40% | y = 0.025831 x - 1.0137 |
| 45% | y = 0.021752 x - 0.80945 |
| 50% | y = 0.018322 x - 0.7054 |
| 55% | y = 0.015539 x - 0.70155 |

The correction factors derived in this manner were used to recalibrate the measured glucose level with respect to the whole blood having a 40% hematocrit level, resulting in the biosensors that can provide hematocrit-independent glucose concentrations. The device reads the speed of sample introduction first, and then determines the level of hematocrit in the blood sample. And, the device takes advantage of the corresponding calibration curves to determine the level of glucose from the measured currents. Table 3 shows the results of the experiment carried out as outlined above.

Table 3. Glucose concentration in whole blood

| Hematocrit % | Glucose YSI2300 (mg/dL) | Hematocrit corrected (mg/dL) |
| --- | --- | --- |
| 30% | 111 | 117 |
|  | 202 | 186 |
|  | 381 | 392 |
| 35% | 138 | 141 |
|  | 200 | 207 |
|  | 276 | 277 |
| 40% | 107 | 112 |
|  | 196 | 195 |
|  | 266 | 264 |
| 45% | 103 | 105 |
|  | 190 | 189 |

(continued)

| Hematocrit % | Glucose YSI2300 (mg/dL) | Hematocrit corrected (mg/dL) |
|---|---|---|
| | 367 | 363 |
| 50% | 102 | 107 |
| | 142 | 143 |
| | 253 | 256 |
| 55% | 125 | 144 |
| | 241 | 240 |
| | 332 | 331 |

The sample introducing speed was measured with the fluidity-determining electrode and the calibration curves of Table 2 were used to estimate the glucose level in whole blood.

The fluidity-determining electrode also discriminated the blood samples of unusual fluidity, i.e., samples with too-high or too-low hematocrit levels and the fouled introduction of blood samples due to the formation of air bubble. In such cases, a reading device may be programmed to issue a warning message or an error code for the measurement.

Experimental Example 4: Reduced Hematocrit Interfering component by Fatty Acid-Containing Reagent Layer

Biosensor strips were prepared as in Example 4. Heparinized whole blood samples were centrifuged to separate the plasma and corpuscles which were remixed to obtain the blood samples of three different hematocrit levels of 20, 40 and 60 %. The effect of hematocrits on the glucose measurement was evaluated at three different glucose concentrations using the biosensors prepared with the reagent layers of Examples 1 and 2. The results are listed in Table 4 and 5.

Table 4. Hematocrit effect on the glucose measurement with the biosensors prepared with the reagent layer of Example 1

| Sample | 1 | | | 2 | | | 3 | | |
|---|---|---|---|---|---|---|---|---|---|
| Hematocrit level | 20 | 40 | 60 | 20 | 40 | 60 | 20 | 40 | 60 |
| YSI Glucose level (mg/dL) | 137 | 126 | 113 | 264 | 238 | 228 | 389 | 377 | 339 |
| Example 1 Reagent based biosensor | 175 | 125 | 88 | 365 | 231 | 146 | 544 | 369 | 114 |
| Bias % relative to 40 % hematocrit level* | 29 | 0 | -22 | 42 | 0 | -34 | 43 | 0 | -66 |
| * % Bias relative to 40 % hematocrit level = {(glucose level by the biosensor/glucose level by YSI)/(glucose level by the biosensor at 40 % hematocrit/ glucose level by YSI at 40 % hematocrit) - 1)x100 | | | | | | | | | |

Table 5. Hematocrit effect on the glucose measurement with the biosensors prepared with the reagent layer of Example 2

| Sample | 1 | | | 2 | | | 3 | | |
|---|---|---|---|---|---|---|---|---|---|
| Hematocrit level (%) | 20 | 40 | 60 | 20 | 40 | 60 | 20 | 40 | 60 |
| YSI Glucose level (mg/dL) | 120 | 111 | 107 | 212 | 199 | 191 | 435 | 398 | 374 |

(continued)

| Sample | 1 | | | 2 | | | 3 | | |
|---|---|---|---|---|---|---|---|---|---|
| Example 2 Reagent-based biosensor | 133 | 114 | 99 | 241 | 201 | 185 | 423 | 382 | 334 |
| Bias % relative to 40 % hematocrit level* | 8 | 0 | -10 | 13 | 0 | -4 | 1 | 0 | -7 |

The results summerized in table 5 show that the biosensor based on example 2 reagents exhibits substantially reduced interfering responses to varying hematocrit levels(from 20% to 60%), whose measurement biases are less than 10% relative to 40% hematocrit level.

As described hereinbefore, the measuring method according to the present invention can substantially reduce the bias arising from hematocrits, taking advantage of the information on sample fluidity in addition to decreasing the possibility of obtaining false information by excluding the blood sample of abnormally high or low fluidity. Further, the biosensor itself can detect the change in blood sampling speed caused by the aging thereof, thereby providing the information about quality control upon manufacture. Moreover, the measuring method of the present invention is advantageous in that a blood sample can be introduced fast and constantly, without being pretreated and accurately analyzed for blood glucose levels within seconds and preferably within 5 sec.

Furthermore, the biosensor, provided with the sample-introducing part 100 for allowing a sample to be introduced fast and constantly without pretreatment and with the fluidity-determining electrode 107 capable of detecting the fluidity of whole blood samples, has a simple structure and can be easily fabricated. 0.1-0.7 $\mu$l of a sample can be introduced constantly into the biosensor without pretreatment. The electrodes of the biosensor show excellent reproductivity. The biosensor is based on a conserve-type, thin-layer electrochemical cell structure in which the working electrode 104 and the auxiliary electrode 105 face each other symmetrically or asymmetrically, with a several hundreds $\mu$m gap set between the electrodes. In such an electrode structure, the current reaches a steady state within 2 sec due to the cycling effect of the redox reactions formed by an enzyme, a substrate contained in the sample, and an electron transfer mediator. In this regard, the reagent layer formed on either the working electrode or the auxiliary electrode is readily dissolved by the sample introduced through the sample channel. Hexaamineruthenium (III) chloride used in the present invention can transfer electrons tens of times faster than can Fe-based electron transfer mediators and is readily dissolved. In addition, the reagent layer composition employed in the biosensor can substantially reduce the measurement bias arising from hematocrits, thereby excluding the influence of interfering components, electrode-activating components, and hematocrits.

**Claims**

1. A method for measuring blood glucose levels, using an electrochemical biosensor provided with a converse-type thin layer electrochemical cell, said converse-type thin layer electrochemical cell comprising:

a working electrode (104) formed on a flat insulating substrate (400);
an auxiliary electrode (105) formed on a separate flat insulating substrate (300) so as to face the working electrode (104);
a fluidity-determining electrode (107), formed at a predetermined distance from the working electrode (104) on the flat insulating substrate (400, 300) used for the working electrode (104) or the auxiliary electrode (105);
an adhesive spacer (200), provided with a sample-introducing part (100) having a micro-passage, for spatially separating the working electrode (104) and the auxiliary electrode (105) by being interposed therebetween;
an electrode connector (106), printed with a thick conductive material on a portion of the auxiliary electrode (105), for three-dimensionally connecting the working electrode (104) to the auxiliary electrode (105); and
a reagent layer containing an electron transfer mediator and an oxidation enzyme, said method comprising the steps of:

(1) introducing a blood sample into a sensor strip-inserted reading device;
(2) applying predetermined respective potential differences between the working electrode (104) and the auxiliary electrode (105) and between the fluidity-determining electrode (107) and the auxiliary electrode (105);

(3) causing a first change in current between the working electrode (104) and the auxiliary electrode (105) so as to allow these electrodes to have the same voltage, as the blood sample is introduced;

(4) detecting the flow of the blood sample with the fluidity-determining electrode (107) to cause a second change in current between the auxiliary electrode (105) and the fluidity determining electrode (107) to adjust voltages between the auxiliary electrode (105) and the fluidity-determining electrode (107) into the same value, thereby providing information about the time difference from the change detected by the working electrode (104);

(5) sufficiently mixing the reagent layer with the blood sample to apply a predetermined voltage between the working electrode (104) and the auxiliary electrode (105) to cause cycling reactions within the converse-type thin layer electrochemical cell; and

(6) determining the level of glucose in the blood sample on the basis of the time information obtained in the step (4) and the steady-state current obtained in the step (5).

2. The method according to claim 1, wherein the blood sample of the step (1) ranges in volume from 0.1 to 0.7 $\mu$l and is introduced into the sensor strip without being pretreated.

3. The method according to claim 1, wherein the potential differences of the step (2) are caused by an electrical change between the working electrode (104) and the auxiliary electrode (105) and between the fluidity-determining electrode (107) and the auxiliary electrode (105) upon applying a direct current, a low- or high-frequency alternating current, a high impedance, or a pulse selected from among square waves, pyramidal waves, half sinewaves, and Gaussian waves.

4. The method according to claim 1, wherein the electrical change is attributed to a change in voltage, current, impedance or capacitance.

5. The method according to claim 1, wherein the sample-introducing part of the biosensor has therein a passage ranging in width from 0.5 to 2 mm and in height from 50 to 250 $\mu$m, thereby facilitating the introduction of the blood sample.

6. The method acccording to claim 1, wherein both the blood sample and the reagent layer are restrained from undergoing redox reactions in the step (3) when the working electrode (104) and the auxiliary electrode (105) are controlled to have the same voltage.

7. The method according to claim 1, wherein the reagent layer containing the enzyme and the electron transfer mediator is formed on either the working electrode (104) or the auxiliary electrode (105).

8. The method according to claim 7, wherein glucose oxidase or glucose dehydrogenase is used as the enzyme.

9. The method according to claim 7, wherein the electron transfer mediator facilitates electron transfer from the enzyme to a final electron acceptor and is hexaamineruthenium (III) chloride.

10. The method according to claim 7, wherein the reagent layer further comprises a fatty acid or its salt and a quaternary ammonium salt to further reduce a hematocrit level-dependent bias.

11. The method according to claim 10, wherein the fatty acid has an alkyl chain of 4 to 20 carbons, preferably wherein the fatty acid is selected from the group consisting of saturated fatty acids having 6 to 12 carbon atoms, more preferably wherein the fatty acid is selected from the group consisting of caproic acid, heptanoic acid, caprylic acid, nonanoic acid, capric acid, undecanoic acid, lauric acid, tridecanoic acid, myristic acid, pentadecanoic acid, palmitic acid, heptadecanoic acid, stearic acid, heptadecanoic acid, stearic acid, nonadecanoic acid, and arachidic acid, and most preferably wherein the fatty acid or its salt is added in an amount from 0.1 to 20 wt% of all solid components.

12. The method according to claim 10, wherein the quaternary ammonium salt is selected from the group consisting of the halide compounds of dodecyltrimethylammonium, myristyltrimethylammonium, cetyltrimethylammonium, octadecyltrimethylammonium, and tetrahexylammonium, and is preferably added in an amount from 0.1 to 30 wt% of all solid components.

**13.** The method according to claim 1, wherein the reagent layer of the biosensor is formed on both or one of the working electrode (104) and the fluidity-determining electrode (107), and the two electrodes are arranged such that the steady-state current time constant is between 0.05 and 8.0, both inclusive.

**Patentansprüche**

**1.** Verfahren zur Messung von Blutglucose-Konzentrationswerten unter Verwendung eines elektrochemischen Biosensors, der mit einer elektrochemischen Dünnschicht-Zelle des Umkehr-Typs versehen ist, wobei die elektrochemische Dünnschicht-Zelle des Umkehr-Typs umfasst:

- eine Arbeitselektrode (104), die auf einem flachen, isolierenden Substrat (400) gebildet ist;
- eine Hilfselektrode (105), die auf einem getrennten flachen isolierenden Substrat (300) gebildet ist, so dass sie der Arbeitselektrode (104) gegenüber liegt;
- eine Fluiditäts-Bestimmungselektrode (107), die in einer vorbestimmten Entfernung von der Arbeitselektrode (104) auf dem flachen isolierenden Substrat (400, 300) gebildet ist, das für die Arbeitselektrode (104) oder die Hilfselektrode (105) verwendet wird;
- einen Klebe-Spacer (200), der mit einem Proben-Einführ-Teil (100), das eine Mikropassage aufweist, versehen ist, zum räumlichen Trennen der Arbeitselektrode (104) und der Hilfselektrode (105), indem er zwischen diesen angeordnet ist;
- ein Elektroden-Verbindungselement (106), das mit einem dicken leitfähigen Material auf einen Abschnitt der Hilfselektrode (105) gedruckt ist, zum dreidimensionalen Verbinden der Arbeitselektrode (104) mit der Hilfselektrode (105); und
- eine Reagenz-Schicht, die einen Elektronen-Transfer-Mediator und ein Oxidationsenzym enthält, wobei das Verfahren die folgenden Schritte umfasst:

(1) Einführen einer Blutprobe in eine Ablese-Vorrichtung mit einem eingesetzten Sensor-Streifen;
(2) Aufbringen jeweiliger vorbestimmter Potential-Differenzen zwischen der Arbeitselektrode (104) und der Hilfselektrode (105) und zwischen der Fluiditäts-Bestimmungselektrode (107) und der Hilfselektrode (105);
(3) Hervorrufen einer ersten Änderung des Stroms zwischen der Arbeitselektrode (104) und der Hilfselektrode (105), um zu ermöglichen, dass diese Elektroden dieselbe Spannung haben, wenn die Blutprobe eingeführt wird;
(4) Messen der Strömung der Blutprobe mit der Fluiditäts-Bestimmungselektrode (107) unter Erzeugen einer zweiten Änderung des Stroms zwischen der Hilfselektrode (105) und der Fluiditäts-Bestimmungselektrode (107) unter Anpassen der Spannungen zwischen der Hilfselektrode (105) und der Fluiditäts-Bestimmungselektrode (107) auf denselben Wert, wodurch man Information über den Zeitunterschied von der Änderung bereitstellt, die durch die Arbeitselektrode (104) gemessen wurde;
(5) ausreichendes Mischen der Reagenz-Schicht mit der Blutprobe unter Anlegen einer vorbestimmten Spannung zwischen der Arbeitselektrode (104) und der Hilfselektrode (105) unter Erzeugen von Kreisreaktionen innerhalb der elektrochemischen Dünnschicht-Zelle des Umkehr-Typs; und
(6) Bestimmung des Konzentrationswerts von Glucose in der Blutprobe auf der Basis der Zeitinformation, die in dem Schritt (4) erhalten wurde, und des Stationärzustands-Stroms, der in Schritt (5) erhalten wurde.

**2.** Verfahren nach Anspruch 1, worin das Volumen der Blutprobe von Schritt (1) im Bereich von 0,1 bis 0,7 µl liegt und dieses in den Sensorstreifen eingeführt wird, ohne vorbehandelt zu sein.

**3.** Verfahren nach Anspruch 1, worin die Potential-Differenzen von Schritt (2) hervorgerufen werden durch eine elektrische Änderung zwischen der Arbeitselektrode (104) und der Hilfselektrode (105) und zwischen der Fluiditäts-Bestimmungselektrode (107) und der Hilfselektrode (105) bei Anlegen eines Gleichstroms, eines Nieder- oder Hochfrequenz-Wechselstroms, einer hohen Impedanz oder eines Impulses, der gewählt ist aus Quadratwellen, Pyramiden-Wellen, Halb-Sinuswellen und Gauss'schen Wellen.

**4.** Verfahren nach Anspruch 1, worin die elektrische Änderung einer Änderung der Spannung, des Stroms, der Impedanz oder der Kapazität zugeschrieben wird.

**5.** Verfahren nach Anspruch 1, worin das Proben-Einführteil des Biosensors eine Passage aufweist, deren Breite im Bereich von 0,5 bis 2 mm liegt und deren Höhe im Bereich von 50 bis 250 µm liegt, wodurch die Einführung der Blutprobe erleichtert wird.

**6.** Verfahren nach Anspruch 1, worin unterdrückt wird, dass sowohl die Blutprobe als auch die Reagenz-Schicht Redox-Reaktionen in Schritt (3) eingehen, wenn kontrolliert wird, dass die Arbeitselektrode (104) und die Hilfselektrode (105) dieselbe Spannung aufweisen.

**7.** Verfahren nach Anspruch 1, worin die Reagenz-Schicht, die das Enzym und den Elektronen-Transfer-Mediator enthält, entweder auf der Arbeitselektrode (104) oder der Hilfselektrode (105) gebildet wird.

**8.** Verfahren nach Anspruch 7, worin Glucoseoxidase oder Glucosedehydrogenase als Enzym verwendet wird.

**9.** Verfahren nach Anspruch 7, worin der Elektronentransfer-Mediator einen Elektronen-Transfer von dem Enzym zu einem End-Elektronen-Akzeptor erleichtert und Hexaaminruthenium(III)-chlorid ist.

**10.** Verfahren nach Anspruch 7, worin die Reagenz-Schicht weiter eine Fettsäure oder ihr Salz und ein quaternäres Ammoniumsalz umfasst, um weiter eine Hematokrit-Wert-abhängige Belastung zu reduzieren.

**11.** Verfahren nach Anspruch 10, worin die Fettsäure eine Alkylkette von 4 bis 20 Kohlenstoffen aufweist, vorzugsweise worin die Fettsäure gewählt ist aus der Gruppe, die besteht aus gesättigten Fettsäuren mit 6 bis 12 Kohlenstoff-Atomen, noch mehr bevorzugt worin die Fettsäure gewählt ist aus der Gruppe, die besteht aus Capronsäure, Heptansäure, Caprylsäure, Nonansäure, Caprinsäure, Undecansäure, Laurinsäure, Tridecansäure, Myristinsäure, Pentadecansäure, Palmitinsäure, Heptadecansäure, Stearinsäure, Nonadecansäure und Arachinsäure, und am meisten bevorzugt worin die Fettsäure oder ihr Salz in einer Menge von 0,1 bis 20 Gew.-% aller festen Komponenten zugesetzt wird.

**12.** Verfahren nach Anspruch 10, worin das quaternäre Ammoniumsalz gewählt ist aus der Gruppe, die besteht aus den Halogenid-Verbindungen von Dodecyltrimethylammonium, Myristyltrimethylammonium, Cetyltrimethylammonium, Octadecyltrimethylammonium und Tetrahexylammonium und vorzugsweise in einer Menge von 0,1 bis 30 Gew.-% aller festen Komponenten zugesetzt wird.

**13.** Verfahren nach Anspruch 1, worin die Reagenz-Schicht des Biosensors gebildet wird auf beiden Elektroden oder einer der Elektroden der Arbeitselektrode (104) und Fluiditäts-Bestimmungselektrode (107), und die beiden Elektroden so angeordnet sind, dass die Stationär-Zustands-Strom-Zeit-Konstante zwischen den Werten 0,05 und 8,0 (beide Werte eingeschlossen) liegt.

## Revendications

**1.** Procédé pour mesurer des niveaux de glycémie, en utilisant un biocapteur électrochimique, pourvu d'une cellule électrochimique à couche mince de type réciproque, ladite cellule électrochimique à couche mince de type réciproque comprenant :

- une électrode de travail (104) formée sur un substrat isolant plat (400) ;
- une électrode auxiliaire (105) formée sur un substrat isolant plat (300) séparé de manière à être placée face à l'électrode de travail (104) ;
- une électrode déterminant la fluidité (107), formée à une distance prédéterminée de l'électrode de travail (104) sur le substrat isolant plat (400, 300) utilisé pour l'électrode de travail (104) ou l'électrode auxiliaire (105) ;
- un écarteur adhésif (200), équipé d'une pièce d'introduction d'un échantillon (100) présentant un micro-passage, pour séparer dans l'espace l'électrode de travail (104) et l'électrode auxiliaire (105) en étant interposée entre celles-ci ;
- un connecteur d'électrode (106), imprimé avec un matériau conducteur épais sur une partie de l'électrode auxiliaire (105), pour une connexion tridimensionnelle de l'électrode de travail (104) à l'électrode auxiliaire (105) ; et
- une couche de réactif contenant un médiateur de transfert d'électrons et une enzyme d'oxydation, ledit procédé comprenant les étapes :

(1) introduire un échantillon sanguin dans un dispositif lecteur avec une bande sensible insérée ;
(2) appliquer des différences de potentiel prédéterminées respectivement entre l'électrode de travail (104) et l'électrode auxiliaire (105) et entre l'électrode de détermination de la fluidité (107) et l'électrode auxiliaire (105) ;

(3) de provoquer un premier changement de courant entre l'électrode de travail (104) et l'électrode auxiliaire (105) de manière à permettre à ces électrodes de présenter le même voltage lorsque l'échantillon sanguin est introduit ;

(4) détecter le flux de l'échantillon sanguin avec l'électrode de détermination de la fluidité (107) afin de provoquer un deuxième changement de courant entre l'électrode auxiliaire (105) et l'électrode de détermination de la fluidité (107) afin d'ajuster les voltages entre l'électrode auxiliaire (105) et l'électrode de détermination de la fluidité (107) à la même valeur, fournissant ainsi de l'information sur la différence de temps avec le changement détecté par l'électrode de travail (104) ;

(5) de mélange suffisant de la couche de réactif avec l'échantillon sanguin afin d'appliquer un voltage prédéterminé entre l'électrode de travail (104) et l'électrode auxiliaire (105) pour provoquer des réactions de cyclage dans la cellule électrochimique à couche mince de type réciproque ; et

(6) déterminer le niveau de glucose dans l'échantillon sanguin sur base de l'information de temps obtenue dans l'étape (4) et du courant à l'état stationnaire obtenu dans l'étape (5).

2.  Procédé selon la revendication 1, dans lequel l'échantillon sanguin de l'étape (1) présente un volume dans la plage de 0,1 à 0,7 μl et est introduit dans la bande sensible sans être prétraité.

3.  Procédé selon la revendication 1, dans lequel les différences de potentiel de l'étape (2) sont provoquées par un changement électrique entre l'électrode de travail (104) et l'électrode auxiliaire (105) et entre l'électrode déterminant la fluidité (107) et l'électrode auxiliaire (105) après l'application d'un courant direct, d'un courant alternatif à fréquence basse ou élevée, d'une impédance élevée, ou d'une impulsion, choisie parmi les ondes carrées, pyramidales, semi-sinusoïdales et gaussiennes.

4.  Procédé selon la revendication 1, dans lequel le changement électrique est attribué à un changement dans le voltage, le courant, l'impédance ou la capacitance.

5.  Procédé selon la revendication 1, dans lequel la pièce d'introduction de l'échantillon du biocapteur présente un passage avec une largeur dans la plage de 0,5 à 2 mm et une hauteur de 50 à 250 μm, facilitant ainsi l'introduction de l'échantillon sanguin.

6.  Procédé selon la revendication 1, dans lequel à la fois l'échantillon sanguin et la couche de réactif sont empêchés de subir des réactions rédox dans l'étape (3) lorsque l'électrode de travail (104) et l'électrode auxiliaire (105) sont contrôlées pour présenter le même voltage.

7.  Procédé selon la revendication 1, dans lequel la couche de réactif contenant l'enzyme et le médiateur de transfert d'électrons est formée sur l'électrode de travail (104) ou l'électrode auxiliaire (105).

8.  Procédé selon la revendication 7, dans laquelle la glucose oxydase ou la glucose déshydrogénase est utilisée comme enzyme.

9.  Procédé selon la revendication 7, dans lequel le médiateur de transfert d'électrons facilite le transfert d'électrons de l'enzyme à un accepteur d'électrons final et est le chlorure d'hexaamine-ruthénium (III).

10. Procédé selon la revendication 7, dans lequel la couche de réactif comprend en outre un acide gras ou son sel et un sel d'ammonium quaternaire pour réduire davantage une erreur systématique dépendant du niveau d'hématocrites.

11. Procédé selon la revendication 10, dans lequel l'acide gras présente une chaine alkyle de 4 à 20 carbones, de préférence dans lequel l'acide gras est choisi dans le groupe constitué par des acides gras saturés présentant 6 à 12 atomes de carbone, plus préférablement dans lequel l'acide gras est choisi dans le groupe constitué par l'acide caproïque, l'acide heptanoïque, l'acide caprylique, l'acide nonanoïque, l'acide caprique, l'acide undécanoïque, l'acide laurique, l'acide tridécanoïque, l'acide myristique, l'acide pentadécanoïque, l'acide palmitique, l'acide heptadécanoïque, l'acide stéarique, l'acide nonadécanoïque, et l'acide arachidique, et le plus préférablement, dans lequel l'acide gras ou son sel est ajouté en une quantité de 0,1 à 20% en poids de tous les composants solides.

12. Procédé selon la revendication 10, dans lequel le sel d'ammonium quaternaire est choisi dans le groupe constitué par les composés halogénés de dodécyltriméthylammonium, de myristyltriméthylammonium, de cétyltriméthylammonium, d'octadécyltriméthylammonium, et de tétrahexylammonium, et est de préférence ajouté en une quantité de 0,1 à 30% en poids de tous les composants solides.

13. Procédé selon la revendication dans lequel la couche de réactif du biocapteur est formée sur les deux ou l'une parmi l'électrode de travail (104) et l'électrode de détermination de la fluidité (107), et les deux électrodes sont agencées de manière telle que la constante de temps du courant à l'état stationnaire est entre 0,05 et 8,0, les deux valeurs étant comprises.

【Figure 1】

【Figure 2】

【Figure 3a】

【Figure 3b】

【Figure 4a】

【Figure 4b】

【Figure 5】

【Figure 6】

【Figure 7】

【Figure 8】

a: Glucose
b: Glucose + acetaminophene (660mM)
c: Glucose + ascorbic acid (570mM)
d: Glucose + uric acid (916mM)

【Figure 9】

【Figure 10】

【Figure 11】

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 1134461 A
- JP 2000338076 B
- US 5658444 A
- US 6241862 B1
- WO 0157510 A2
- EP 1186778 A
- US 2004004821 A

### Non-patent literature cited in the description

- **G. CUI et al.** *Electroanalysis,* 2001, vol. 13, 224-228
- **G. CUI et al.** *Talanta,* 2001, vol. 54, 1105-1111
- **E. K. BAUMAN et al.** *Analytical Chemistry,* 1965, vol. 37, 1378
- **K. B. OLDHAM.** Microelectrodes: Theory and Applications. Kluwer Academic Publishers, 1991